(19) **Europäisches Patentamt / European Patent Office / Office européen des brevets**

(11) **EP 4 495 224 A1**

(12) **EUROPEAN PATENT APPLICATION**
published in accordance with Art. 153(4) EPC

(43) Date of publication:
**22.01.2025 Bulletin 2025/04**

(21) Application number: **23769379.1**

(22) Date of filing: **13.03.2023**

(51) International Patent Classification (IPC):
**C12N 1/00** $^{(2006.01)}$

(52) Cooperative Patent Classification (CPC):
**C12N 1/00**

(86) International application number:
**PCT/BR2023/050086**

(87) International publication number:
**WO 2023/173189 (21.09.2023 Gazette 2023/38)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **15.03.2022 BR 102022004731**

(71) Applicant: **Yessinergy Holding S.A.**
**13049-254 Campinas (BR)**

(72) Inventors:
- **ABOU NEHMI FILHO, Victor**
  **05350-000 SÃO PAULO (BR)**
- **BUENO DA SILVA, Juliana**
  **13451-010 Santa Bárbara d´Oeste (BR)**

(74) Representative: **Sanchez Benito, Arturo**
**Jacobacci & Partners, S.L.**
**Calle Zurbano 76, planta 7**
**28010 Madrid (ES)**

(54) **PROCESS FOR PRODUCING AND PURIFYING FRUCTOOLIGOSACCHARIDES AND PRODUCT OBTAINED BY SAID PROCESS**

(57)     This invention relates to a process for the production and purification of fructooligosaccharides (FOS) and the product obtained by such process having low or no residue of glucose, or fructose, or sucrose, with the FOS having a different ratio of oligomers (GF2, GF3, GF4, GF5 and GF6) and their isomers, to be used in animal and human nutrition due to their superior properties of modulating the intestinal microbiota and direct and indirect immunomodulation.

The process for producing and purifying fructooligosaccharides according to the invention employs at least two microbes or yeasts, simultaneously or separately, with one of the microbes or yeasts being unable to produce extracellular invertase in a medium containing sucrose, in order to convert sucrose into fructooligosaccharides (FOS), with total consumption of glucose from the medium and evaporation of alcohol or ethanol during drying of the final product.

FIGURE 3

EP 4 495 224 A1

**Description**

FIELD OF APPLICATION

The present invention relates to a process for the production and purification of fructooligosaccharides (FOS) and the product obtained by such a process with low or no residue of glucose, or fructose, or sucrose, with FOS having a different proportion of oligomers (GF2, GF3, GF4, GF5 and GF6) and their isomers, to be used in animal and human nutrition due to their superior properties of modulating the intestinal microbiota and direct and indirect immunomodulation.

PRIOR ART

FOS or oligofructose are one of the most popular prebiotic oligosaccharides studied and used in human and animal nutrition, as they have a recognized bifidogenic effect. Since, after being fermented, preferably by bifidobacteria and lactobacilli present in the intestinal tract, short-chain fatty acids are produced, such as acetic, propionic, butyric and lactic acids. These compounds indirectly promote modulation in the intestinal microbiota, through acidification of the environment, favoring the growth of beneficial bacteria to the detriment of putrefactive bacteria that cause enteritis, such as those of the genera *Salmonella, Eschirichia coll, Clostridium, Campylobacter* and other pathogens.

In addition to decreasing intestinal pH, these organic acids are absorbed by enterocytes and used as a source of energy, increasing the production of mucin (protective mucosa of the intestinal epithelium), promoting the increase in intestinal villi and improving the absorption of nutrients, they strengthen the "tight junctions" promoting greater integrity of the intestinal epithelium, in addition to promoting metabolic regularization, controlling blood levels of glycemia, triglycerides and cholesterol.

Modulation of the microbiota, in addition to contributing to eubiosis and balancing a diversity of hormones produced in the intestine, today considered the second brain, promotes a secondary immunomodulatory effect, since beneficial microorganisms activate T cells, which produce cytokines that recruit cells to combat inflammatory diseases and pathogens, such as interleukins IL-1, IL-2, IL-4, IL-6, IL-10, and others. They also act on the adaptive immune system by increasing the production of IgA, IgG and IgM antibodies by B cells.

More recent studies show the role of prebiotic oligosaccharides, such as long-chain FOS (IcFOS) and short-chain GOS (scGOS), acting directly on the immune system, which are recognized by the "tool like receptors" (TLR-4) and the production of interleukin 10, thus demonstrating their direct action, i.e., even though they are not fermented or consumed by probiotic bacteria, even in the earliest part of the intestine, they exert a significant influence on the innate immune system.

FOS can be found in many plants and foods such as chicory (*Chichorium intybus*), artichokes, Jerusalem artichokes, yacon, onions, bananas, tomatoes, garlic, asparagus, agave americana, honey and sugar cane. They are reserve carbohydrates and have osmoregulatory action in plants. Its formation is due to environmental conditions and stage of development, and low temperatures induce the accumulation of fructans in the leaves and roots.

They are also produced by many microorganisms, such as *Aspergillus, Aureobasidium sp., Fusarium, Penicillium, Gliocladium virens, Sporutrichum thermophile, Scopulariopsis brevicaulis, Rhodothorula, Klyveromyces marxianus, Xanthoplylomyces dendrohous, Schwanniomyces occidentalis, Saccharomyces cerevisiae, Bacillus e Zymomonas mobilis.*

Both in plants and through microorganisms, its formation is due to hydrolytic enzymes (EC.3.2.1.26) and transferases (EC.2.4.1.9), such as fructofuranosidases and fructosyltransferases.

FOS differ according to the chain size and the bond between the fructose molecules, depending on their formation enzyme. According to IUPAC-IUB (International Union of Pure and Applied Chemistry), oligosaccharides are defined as saccharides or carbohydrates that contain 3 to 10 monosaccharide units in their chain. Therefore, fructose oligomers can contain up to 10 fructose molecules in their composition. However, many authors prefer the definition that they are molecules that contain up to 10 degrees of polymerization. Above this value, they are called fructan polysaccharides or "fructans", differing between levans and inulin, whose molecules can contain up to 60 fructose units in their chain.

Fructose chains can contain 1F and 6G and 6F type bonds. The first type has fructose units linked by osidic bonds of the $\beta$-2,1 type. The 6G are called neo-fructooligosaccharides (NeoFOS), they have osidic bonds of the $\beta$-2,6 type, between fructose and glucose units. As for type 6F, they have osidic bonds of the $\beta$-2,6 type between fructose units.

The best-known FOS synthesis process follows the mathematical model for the action of fructosyltransferase from *Aureobasidium pullulans.*

$$GFn + GFn \rightarrow GFn\text{-}1 + GFn\text{+}1 \quad n = 1 - 3$$

(G: glucose, F: fructose, GF: sucrose)

This mechanism is divided into three reactions:

$$2 \, GF \rightarrow GF2 + G \qquad (1)$$

$$2 \, GF2 \rightarrow GF3 + GF \qquad (2)$$

$$2 \, GF3 \rightarrow GF4 + GF3 \qquad (3)$$

In this model, excess glucose acts as an inhibitor of the fructosyltransferase enzyme, thus exerting the effect of negative feedback, i.e., once present in a certain quantity in the reaction medium, the reaction is then stopped. As a result, the FOS synthesis process, in the prior art, has a maximum yield of 60%, leaving sucrose remaining and reaching very high levels of residual glucose, representing up to 30% of the total components, which makes spray drying unfeasible.

Classic example of a standard FOS reaction using the fructosyltransfererase enzyme:

$$[GF] \rightarrow [G] + [F] + [GF] + [GF2 + GF3 + GF4]$$

$$40\% \rightarrow 12\% + 1\% + 3\% + 24\%$$

$$\text{Yield} = [GF2 + GF3 + GF4]/[GF] =$$
$$= 24\%/40\% = 60\%$$

As a result, the excessive concentration of glucose in the medium can be noted, preventing the reaction from continuing to occur, and may even form larger oligomers, such as the mathematical example below:

$$2 \, GF \rightarrow GF2 + G \qquad (1)$$

$$2 \, GF2 \rightarrow GF3 + GF \qquad (2)$$

$$2 \, GF3 \rightarrow GF4 + GF3 \qquad (3)$$

$$2 \, GF4 \rightarrow GF5 + GF_4 \qquad (4)$$

$$2 \, GF5 \rightarrow GF6 + GF_5 \qquad (5)$$

The present invention proposes and proves the formation of GF5 and GF6, through the simultaneous elimination of glucose together with the synthesis of FOS.

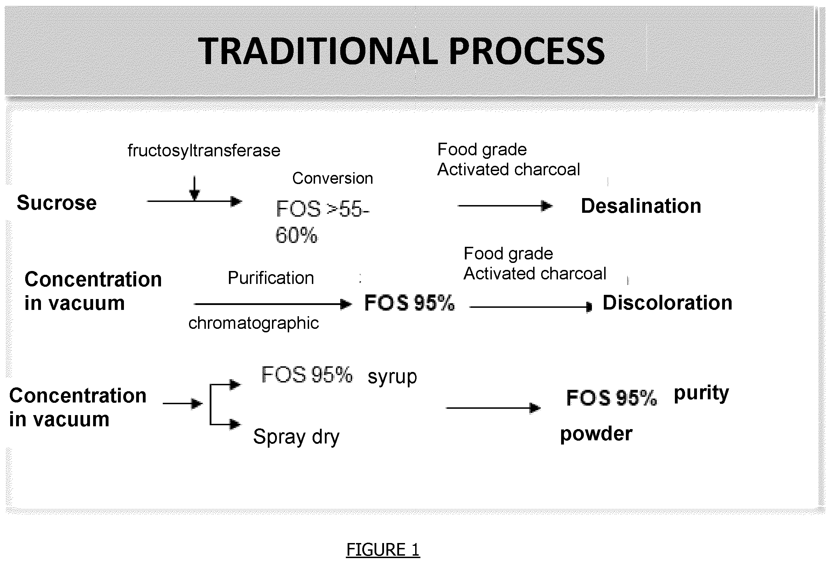

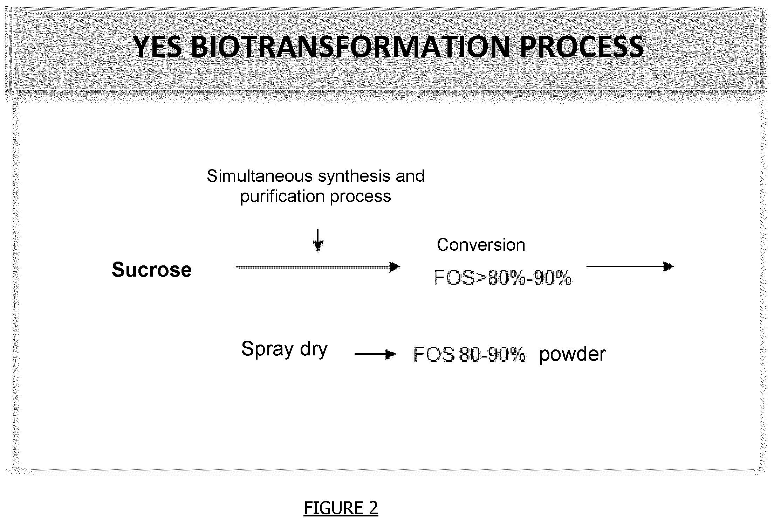

To remove glucose in the final FOS syrup, the method most used in the prior art is the use of chromatographic columns. By size exclusion (ionic sieves), sugars with lower molecular weights, such as glucose, fructose and sucrose, are eliminated until reaching 100% purity of FOS in the purified syrup and 95% purity of FOS in the final product. Figures 1 and 2 show the purification steps of the traditional FOS production process compared to the process of the present invention.

On the other hand, the present invention offers as an alternative thereto, the use of the biotransformation process, by using intact microorganisms, both with the aim of synthesizing FOS and with the aim of purifying it by removing glucose and other residual sugars from the reaction medium. For such, in addition to *Aureobasidium sp.* responsible for the synthesis of FOS, a *Saccharomyces cerevisiae* yeast was used that selectively consumes only glucose and fructose and does not consume sucrose and FOS, i.e., the oligomers with $\alpha,1$-2 and $\beta,2$-1 linkages. As a consequence, the sucrose conversion yield increased to a minimum of 70%, with the presence of residual sucrose not being observed and in the syrup and in the final product the FOS concentration reached minimum values of 80 to 90% since the ethanol was completely evaporated in the spray dryer drying process.

The concentration greater than 3% of glucose in the FOS syrup does not allow the drying process using the spray drying technique to take place. This is due to the low degree of glass transition (t.g.) of low molecular weight sugars, such as glucose and fructose, which does not occur with sugars with a higher degree of polymerization, such as kestose (GF2), nystose (GF3) and fructofuranosylnystosis (GF4). The elimination of glucose by a microbiological process is low cost, when compared to the separation of glucose by chromatography, as is done by the current prior art.

Prior art document JPH 04293494 teaches the effective production of a fructose-containing oligosaccharide or glycoside useful in the field of medicine and food at a low cost by treating sucrose with group fructosyl transferase and invertase-deficient yeast in the presence of an acceptor. Sucrose is dissolved in water in the presence of an acceptor (e.g. lactose), reacted with beta-fructofuranosidase, a fructosyl group transferase produced by *Arthrobacter sp. K-1* and simultaneously with invertase-deficient yeast at pH 6.5-7.0 at 35 °C for 20 hours, the invertase-deficient yeast is removed by filtration with diatomaceous earth, the filtrate is treated with active charcoal at 95°C. C for 1 hour, purified with an ion exchange resin, concentrated and spray dried to give a fructose-containing oligosaccharide comprising lactosylfructoside or a fructose-containing glycoside such as fructosyl stevioside.

However, the present invention differs in that it solved a problem and achieved an unusual result.

PROBLEM SOLVED

The present invention solves the problem of the person skilled in the art of achieving a better yield in the process of obtaining fructooligosaccharides (FOS) and mainly better purity due to the elimination of residues of glucose, or fructose, or sucrose, some by-products that make it very difficult and mainly make the purification conditions of the final product obtained more expensive. Furthermore, the invention optimizes the obtaining of the final product, in a sustainable manner, by eliminating alcohols, particularly ethanol from the final product, during the spray dryer drying stage.

UNUSUAL EFFECT OF THE INVENTION

The present invention, in addition to obtaining a final product with greater purity, employing a more sustainable process and employing more economical equipment and conditions than those employed by the person skilled in the art, also allows obtaining a final product with a greater quantity of long-chain oligomers, such as GF5 and GF6, important to reach the colonies of bifidobacteria and lactobacilli in the most distal part of the large intestine, which are not reached by the short-chain oligomers GF2, GF3 and GF4. This was possible because the two microorganisms (*Aureobasidium* and *Saccharomyces*) are able to live together and act together and simultaneously in the process.

OBJECTIVES

A process for obtaining and purifying fructooligosaccharides (FOS) using yeast was developed, which aimed to present:

- the direct biotransformation of sucrose into FOS, with high conversion rates and yield;
- microbial elimination of glucose;
- microbial elimination of glucose, thanks to the use of *Saccharomyces cerevisiae* yeast which does not produce extracellular invertase, obtained by using the CRISPR Cas9 methodology;
- lower production cost of fructooligosaccharides;
- possibility of direct drying of the syrup containing the fructooligosaccharides obtained;
- production of FOS with different proportions of short-chain oligomers (scFOS such as GF2, GF3, GF4) and with a significant presence of long-chain oligomers (lcFOS such as GF5 and/or GF6) and their isomers;
- possibility of producing FOS with proportions of oligomers more appropriate for each purpose of use, in terms of more effective functional physiological action, greater modulation of the microbiota and greater promotion of eubiosis, in addition to being a direct immunomodulator;
- a more sustainable process than that used by the person skilled in the art.

BRIEF DESCRIPTION OF THE FIGURES

The figures are briefly commented below:

Figure 1: Traditional process for obtaining fructooligosaccharides.
Figure 2: Process for obtaining fructooligosaccharides according to the present invention.
Figure 3: Description of the FOS formation reaction from the sucrose molecule. GF: sucrose, G: glucose, F: fructose and GF2, GF3, GF4: FOS.
Figure 4: indicates A: Standard process or prior art.
Figure 5: indicates B: process referred to in the present invention.
Figure 6: Representative scheme of the SUC2 gene and the genome editing strategy. A) Location of the SUC2 gene on chromosome IX and the design of primers for editing the gene promoter region and consequent deletion thereof using the CRISPR/Cas 9 technique. B) Structure of the SUC2 gene after editing the gene promoter.
Figure 7: Canonical Correlation for fermentative and fecal quality parameters between Negative Control (NC) and prebiotic-containing treatments.

SUMMARY OF THE INVENTION

The present invention relates to a process for the production and purification of fructooligosaccharides using at least two microbes or yeasts, simultaneously or separately, with one of the microbes or yeasts not being able to produce extracellular invertase, in a medium containing sucrose, in order to convert sucrose into fructooligosaccharides (FOS), with total consumption of glucose from the medium and evaporation of alcohol or ethanol during drying of the final product, and final product obtained.

DETAILED DESCRIPTION

The present invention describes a new product containing short-chain fructooligosaccharides, GF2, GF3, GF4 (scFOS) and long, such as GF5 and GF6 (lcFOS) and their isomers, originating from an innovative purification process, whereby the sugars residues, such as glucose and fructose, are eliminated by *Saccharomyces cerevisae* yeasts, instead of traditional removal through chromatographic purification. Furthermore, the yield of the sucrose to FOS conversion process, which in prior art is on the order of 60%, was increased to 70%, since the synthesis and purification of FOS occurred at the same time, preventing the effect of negative feedback that excess glucose has on the performance of the fructosyltransferase enzyme.

The greater proportion of longer chain oligomers provides a greater bifidogenic effect to the FOS product according to the present invention, since the longer chain oligomers reach more distal proportions of the large intestine, thus being fermented in a larger area of the intestinal wall. Since, when there is a high concentration of short-chain oligomers, as occurs in prior art products, their fermentation tends to occur at the beginning of the large intestine, while little FOS reaches the distal part.

The present invention also drastically reduces the cost of producing FOS, since the glucose removal step through purification by chromatographic columns becomes unnecessary, which allows direct drying of the syrup by spray dryer and provides a product in powder form. The invention, object of the present invention, carries out the synthesis and purification through the microbial biotransformation of sucrose into FOS, through enzymes produced by *Aureobasidium pullulans,* and the selective consumption of glucose and fructose through the use of a strain of *Saccharomyces cerevisae* that does not produce extracellular invertase, thus allowing a faster, more effective process and without investment costs in equipment such as the chromatographic column. This cost reduction makes it possible to use this ingredient in the animal nutrition market and in the food industry.

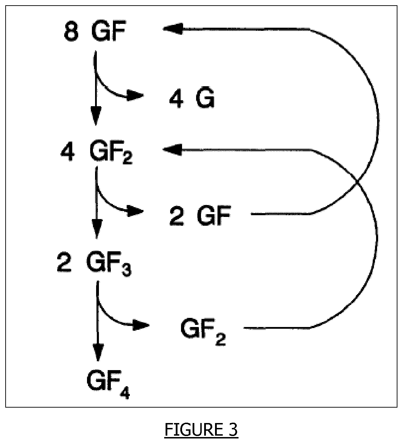

A process for the production and purification of fructooligosaccharides was developed by using a co-propagation of microbial inocula containing *Aureobasidium sp*. and a strain of *Saccharomyces cerevisiae* that does not produce extracellular invertase, which was inoculated in a medium containing high concentrations of sucrose, ranging from 20 to 60% sucrose, for a minimum period of 12 hours, but up to 72 hours, sufficient to convert sucrose into FOS, with immediate consumption of glucose, simultaneously with its release into the medium, according to the reaction illustrated in Figure 3 attached, where GF: sucrose; G: glucose; F: fructose; and GF2, GF3, GF4: FOS.

The increase in glucose concentration in the medium exerts a negative "feedback" effect on the action of the fructosyltransferase enzyme, which means that the FOS synthesis reaction is negatively influenced by the presence of glucose until its concentration comes to a complete standstill. the formation of FOS.

By inhibiting this negative effect of glucose on the action of the enzyme, the present invention makes it possible to increase the conversion of sucrose into FOS. Furthermore, it allows the degree of polymerization of the oligomers formed to be increased, as well as favoring the formation of isomers of the fructooligosaccharides formed. The increase in the reaction yield leads to a decrease in the residual sucrose content, and, mainly, eliminates the glucose residue in the product, making it a product with at least 80% purity. Most of the glucose is transformed into ethanol, which is eliminated by evaporation during drying in the spray drier.

One of the striking characteristics of the product of this invention was the obtaining of long-chain FOS, so that this product is not as long as a fiber, nor as common as the prior art product, thus differing in physiological effects caused in the body: greater bifidogenic effect, greater effect on immunity directly and indirectly.

The greater bifidogenic effect observed in FOS, the product of the present invention, when compared to prior art FOS, is due to the longer chain oligomers (GF5 and GF6) and the larger average size of the other short-chain oligomers (GF2, GF3 and GF4). Longer chain oligomers reach more distal portions of the large intestine, so that better distribution of oligomer proportions allows for more uniform fermentation by good bacteria throughout the entire large intestine. In prior art FOS, with a high concentration of short-chain oligomers (GF2 and GF3), fermentation is uneven, concentrating in the initial part of the large intestine. As a result, the prior art FOS is more likely to cause SIBO (small intestine bacterial overgrowth) events than the product according to the invention.

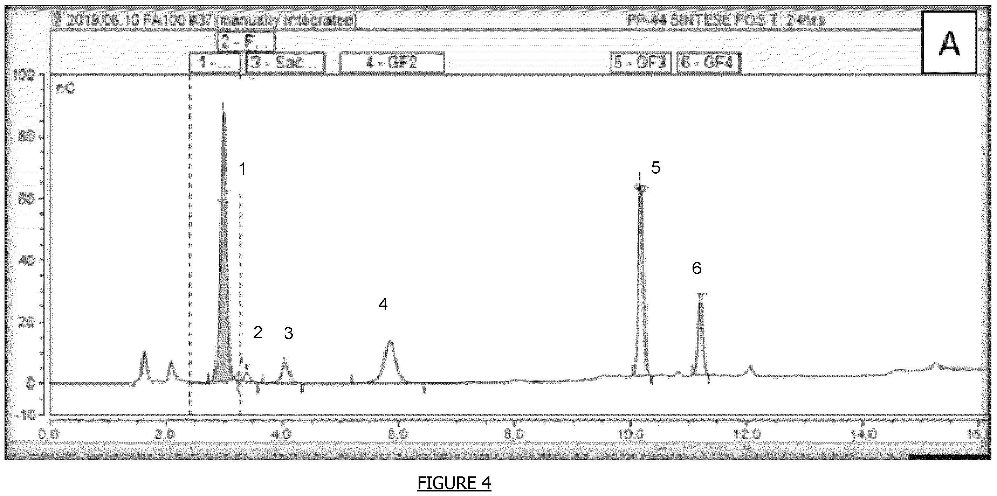

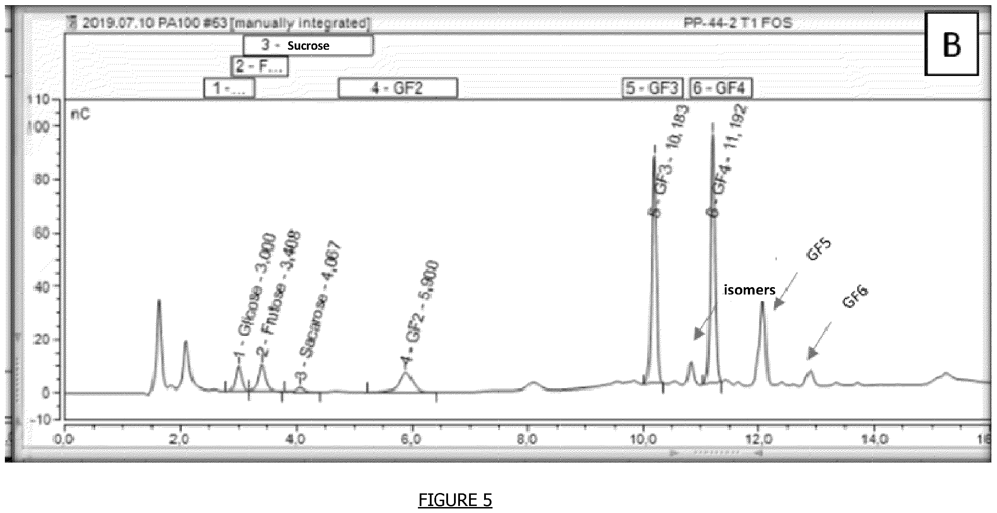

Figures 4 and 5, attached, show the figures of the chromatogram obtained by analyzing the product on an ion chromatograph, model ICS-5000, from Thermofisher, with a Carbopac PA100 column.

In Figure 4, attached, peak (1) in orange represents Glucose - 2,992, peak (2) represents Fructose - 3,400, peak (3) represents Sucrose - 4,050, peak (4) represents GF2 - 5,850, peak (5) represents GF3 - 10,175 and peak (6) represents GF4 - 11,192.

In Figure 5, attached, peak (1) in orange represents Glucose - 3,000, peak (2) represents Fructose - 3,408, peak (3) represents Sucrose - 4,067, peak (4) represents GF2 - 5,900, peak (5) represents GF3 - 10,183, peak (6) represents GF4 - 11,192, peak (7) represents isomers, peak (8) represents GF5 and peak (9) represents GF6.

Figure 4 (where A: Standard process, B: process referred to in the present invention) illustrates the standard FOS production processes (current prior art) and that of the present invention, Figure 5, in which the presence of GF5 and GF6 and other intermediate isomers, in addition to the clear decrease in glucose.

The present invention deals with a process for producing fructooligosaccharides and a product containing short-chain fructooligosaccharides, GF2, GF3, GF4, long-chain GF5 and/or GF6, their isomers and low concentrations of sucrose, as detailed in Figure 1 and in Figure 2, when comparing the prior art processes and the process of the present invention.

Raw material:

The invention uses various sources of sucrose, which may be sugar from sugar cane, beetroot, sugar cane syrup and molasses, preferably VHP (very high polarization) sugar, which is the most concentrated source of sucrose and the lowest cost known. There is little reference to the direct use of VHP sugar in the transformation, whether biological or enzymatic, of sucrose into FOS. Several studies indicate that the higher the concentration of sucrose in the medium where the enzymatic synthesis of FOS occurs, the higher the rate of conversion of sucrose into FOS. The process, according to the invention, starts with a solution of VHP sugar diluted in water, in a ratio that can vary from 20% to 60% sugar. This solution was inoculated with a culture of *Aureobasidium sp.* and the genetically modified strain of *Saccharomyces cerevisiae* PE-2 *Saccharomyces cerevisiae,* which does not produce extracellular invertase, in an inoculum amount of 10 to 20% (v/v) in the final medium, so that the biotransformation of sucrose into FOS occurs and simultaneous consumption of glucose.

In the present invention, a strain of *Saccharomyces cerevisiae* yeast called PE-2 was used, a wild strain, isolated from a Brazilian industrial sugar and ethanol environment, which was subjected to a simple deletion of the gene related to invertase production, as described below.

1. Deletion of the invertase gene in Pe-2 yeast

The *Saccharomyces cerevisiae* yeast used in the purification step of this invention has low and/or no ability to metabolize sucrose, as well as fructooligosaccharides, which are the products of interest. This inability leads to selective consumption of glucose and fructose in FOS syrup.

According to an embodiment of the invention, for the disruptive mutation of the SUC-2 gene, the genome editing strategy is used, by using the modified CRISPR/Cas 9 technique, where the 1109 bp of the gene was deleted, preventing the functional expression of the encoded protein. Editing is validated through PCR genotyping, growth on sucrose (it is null in the case of edited yeast) and Sanger sequencing.

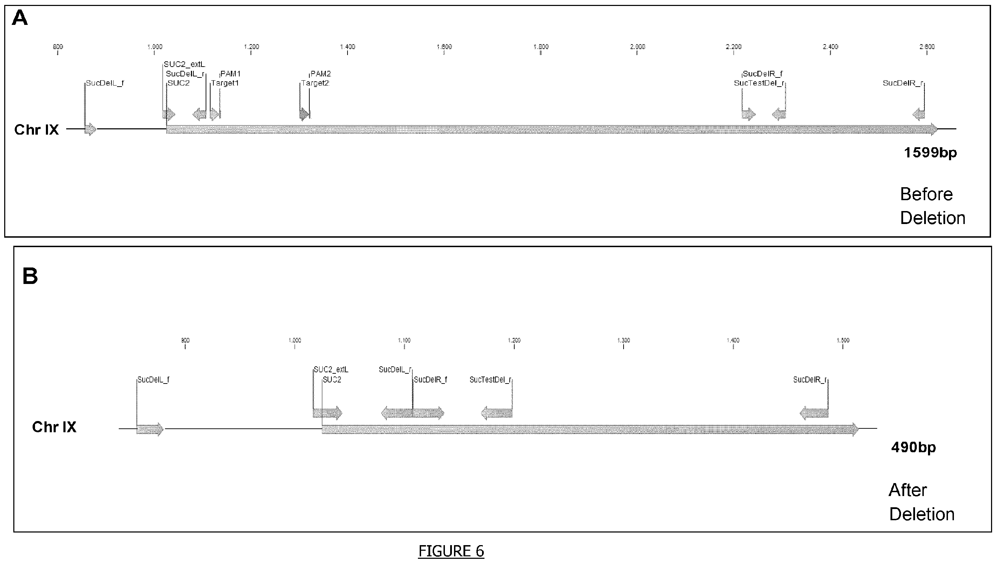

The SUC2 gene is located on Chromosome IX of the *Saccharomyces cerevisiae* genome and contains 1599 base pairs. The SUC2 gene is located in the *Saccharomyces cerevisiae* Pe-2 genome. With the genomic editing described above, it now has 490 base pairs (Figure 6 attached: Representative scheme of the SUC2 gene and the genome editing strategy. A) Location of the SUC2 gene on chromosome IX and the design of primers for genome editing region of the gene promoter and consequent deletion thereof using the CRISPR/Cas 9 technique. B) Structure of the SUC2 gene after editing the gene promoter), completely making its protein expression unfeasible.

Gene manipulation was applied to chromosome 9, where tools were used to further reduce this risk of off-target, which are cleavages at undesirable points in the genome by Cas 9.

According to the literature (Ryan OW, Skerker JM, Maurer MJ et al. Selection of chromosomal DNA libraries using a multiplex CRISPR system. eLife 2014;3:e03703; Jakociunas T, Bonde I, Herrgard M et al. Multiplex metabolic pathway engineering using CRISPR/Cas9 in Saccharomyces cerevisiae. Metab Eng 2015;28:213-22), in small genomes such as *Saccharomyces cerevisiae* it is very unlikely that off-target effects will occur. gRNAs (guide RNAs) were designed for a unique sequence in the genome, which makes it impossible for the Cas9 enzyme to recognize any other PAM (protospacer adjacent motif) sequence in the experiment.

The expression product of the genomic region: with the deletion, there is no expression of the invertase encoded by SUC2, which was verified by the change in phenotype leading to the yeast not growing in medium YP + 2% sucrose and normal growth in medium YP+2% glucose.

2. Co-propagation of the inoculum for FOS synthesis

According to the present invention, it was possible to optimize the propagation of the inoculum in one single step, whereby the *Aureobasidium sp* strain, responsible for the synthesis of FOS, and the *Saccharomyces cerevisiae* PE-2 strain, which does not produce invertase, are propagated together, described above, in one single reactor.

The process can take place separately, however, by conducting it this way, the production process is optimized.

To this end, first prepare a volume of "9X" liters of aqueous medium containing 10 to 20% total reducing sugars (TRS), a volume of "1X" liters of *Aureobasidium* and after 24 hours of growth, the *Saccharomyces cerevisiae* genetically modified is inoculated, which is incubated for another 24 hours, with a temperature set between 32 and 35°C, with 120 rpm of agitation and at least 1 vvm of aeration.

After complete growth of both cultures, "10X" liters of inoculum culture are transferred to "90X" liters of culture medium containing 10 to 20% TRS (total reducing sugars), incubated at a controlled temperature between 32 at 35°C, with 120 rpm of agitation and at least 1 vvm of aeration, for 24 to 48 hours, until complete growth and a minimum population of 5. $10^7$ CFU/mL of each yeast (*Aureobasidium sp* and genetically modified *Saccharomyces cerevisiae*).

3. FOS synthesis and simultaneous glucose consumption.

After growth, as mentioned above, the inoculum is transferred to an aqueous medium of sucrose and/or molasses and/or sugar cane syrup containing 20 to 60% sucrose in the total medium, in a ratio of 10 to 20% of the volume of the aqueous medium.

FOS synthesis occurs in a minimum period of 12 hours. However, the removal of glucose through the consumption of *Sacharomyces* yeast occurs between 12 and 60 hours, a period that varies depending on the initial sucrose concentration of the medium. Therefore, this process occurs between 30 and 35°C, for a period of at least 12 hours, but can last up to 60 hours.

Through this invention, it was possible to increase product purity and yield by converting all or almost all of the sucrose content into FOS, since simultaneous consumption of glucose avoids the "negative feedback" effect of glucose on the activity of the fructosyltransferase enzyme, increasing the rate of conversion of sucrose into FOS.

The ethyl alcohol produced is 100% evaporated during spray drying so that the final product contains high purity.

Table 1 (kinetic of formation of fructooligosaccharides in an initial medium containing 44% sucrose on a wet basis and dry basis, respectively) and table 2 (composition of the prior art product and the present invention), below, demonstrate the selective consumption of glucose and fructose in the syrup and presents the high concentrations of FOS obtained in the simultaneous production and purification process (meaning of the abbreviations: scFOS, short-chain FOS; LcFOS: long-chain FOS):

TABLE 1

| | Carbohydrates % (m/v) | | | | | | | | |
|---|---|---|---|---|---|---|---|---|---|
| Time (h) | Glucose | Fructose | Sucrose | GF2 | GF3 | GF4 | scFOS | LcFOS | FOS total |
| 0 | - | - | 43.2 | - | - | - | - | - | - |
| 12 | 12.8 | 1.1 | 4.4 | 16.5 | 7.9 | 1.0 | 25.4 | - | 25.4 |
| 24 | 8.7 | 1.6 | 3.4 | 10.1 | 11.7 | 4.8 | 26.6 | - | 26.6 |
| 36 | 6.6 | 1.3 | 2.3 | 8.6 | 15.2 | 5.1 | 28.9 | - | 28.9 |
| 48 | 4.4 | 1.3 | 2.2 | 6.8 | 14.7 | 4.8 | 26.3 | 1.2 | 27.5 |
| 60 | 1.2 | 0.5 | 0.5 | 7.0 | 12.9 | 4.5 | 24.4 | 3.3 | 27.7 |
| 72 | 0.0 | 0.0 | 0.4 | 6.9 | 12.9 | 4.1 | 23.9 | 4.0 | 27.9 |
| | Carbohydrates % (m/m) | | | | | | | | |
| Time (h) | Glucose | Fructose | Sucrose | GF2 | GF3 | GF4 | scFOS | LcFOS | FOS total |
| 0 | - | - | 97.1 | - | - | - | - | - | - |
| 12 | 28.8 | 2.4 | 10.4 | 39.1 | 18.6 | 2.3 | 60.0 | - | 60.0 |
| 24 | 19.5 | 3.5 | 9.1 | 26.8 | 30.9 | 12.7 | 70.4 | - | 70.4 |
| 36 | 14.9 | 3.0 | 6.6 | 24.5 | 43.4 | 14.6 | 82.4 | - | 82.4 |
| 48 | 9.9 | 2.9 | 6.5 | 20.3 | 44.0 | 14.5 | 78.8 | 3.6 | 82.4 |

(continued)

| Carbohydrates % (m/m) | | | | | | | | | |
|---|---|---|---|---|---|---|---|---|---|
| Time (h) | Glucose | Fructose | Sucrose | GF2 | GF3 | GF4 | scFOS | LcFOS | FOS total |
| 60 | 2.7 | 1.1 | 1.7 | 22.5 | 41.2 | 14.3 | 78.0 | 10.5 | 88.4 |
| 72 | 0.1 | 0.0 | 1.2 | 22.8 | 42.9 | 13.6 | 79.3 | 13.3 | 92.6 |

In Table 1, one can clearly observe the consumption of glucose simultaneously with the formation of FOS, from 24 hours onwards. This allows a higher yield than the prior art process, which has a yield of 60% in the transformation of sucrose into FOS. The consumption of glucose generates ethanol and, at the same time, the reduction in dry matter, which initially was 45% and at the end of the 72-hour process decreased to 30%.

Through the formation of ethanol, it was possible to obtain high purity of the syrup in FOS. In the composition of the final product, 3 to 10% of supporting drying additives are added, such as silicon oxide or corn starch, so that, if the humidity of the powder in the composition is also taken into account, the purity of the final product decreases by 10% on average.

Table 2 compares the composition of pure FOS, according to the present invention, with prior art FOS, in the case produced by the company Ingredion indicated in table 3. Composition of FOS commercial product

TABLE 2

| | Glucose | Fructose | Sucrose | GF2 | GF3 | GF4 | scFOS | lcFOS | FOS total |
|---|---|---|---|---|---|---|---|---|---|
| FOS Yes | 0,05 | 0,03 | 1,05 | 20,0 | 37,5 | 11,9 | 69,4 | 11,6 | 81,0 |
| Prior art FOS | 0,57 | 0,55 | 3,19 | 45,8 | 33,6 | 12,6 | 92,0 | - | 92,0 |

Composition of pure FOS

TABLE 3

| | Glucose | Fructose | Sucrose | GF2 | GF3 | GF4 | scFOS | lcFOS | FOS total |
|---|---|---|---|---|---|---|---|---|---|
| FOS Yes | 0.1% | 0.0% | 1.3% | 24.6% | 46.3% | 14.7% | 85.7% | 14.3% | 100.0% |
| Prior art FOS | 0.6% | 0.6% | 3.5% | 49.8% | 36.5% | 13.7% | 100.0% | 0.0% | 100.0% |

### 4. Microbial elimination of glucose

The high rate of conversion of sucrose to FOS, as occurred in the present invention, has the disadvantage of releasing large amounts of glucose into the solution in which the synthesis occurs. The presence of a high concentration of glucose, which can exceed 22%, would prevent the FOS syrup from drying using the spray drying technique. The solution found according to the invention was to transform glucose into ethyl alcohol, by using yeast without invertase activity.

The present invention drastically reduces the cost of producing fructooligosaccharides, since the glucose removal step through chromatographic purification becomes unnecessary, it allows direct drying of the FOS syrup (as the product is called before drying) by spray drying and enables its mass use in animal nutrition and the food industry, which are more sensitive to costs. To this end, the biochemical transformation of glucose is carried out by using yeast or a microbe, *Saccharomyces cereviae,* which does not produce extracellular invertase.

### 5. Direct drying of the Syrup

The presence of glucose in concentrations above 3% in the FOS syrup does not allow the direct drying process in the spray drier. This occurs due to the glass transition temperature (Tg) of the compounds that differ from each other. Glucose and fructose have a low t.g. value, not allowing spray drying, which does not occur with sugars with a higher degree of polymerization, such as kestose (GF2), nystose (GF3) and fructofuranosylnystose (GF4). The production of FOS and the elimination of glucose by a microbiological process is characterized by being a low-cost process, when compared to the separation of glucose by chromatography, as is done by the current prior art.

Obtaining the final product:

In the case of the present invention, we chose to use *Aureobasidium sp.* for the biotransformation of sucrose, as the FOS it produces contains only shorter chain oligomers (GF2, GF3 and GF4, GF5 and/or GF6), which are those with the greatest bifidogenic effect.

Because the FOS of the present invention can be partially or totally purified, their concentration can vary from 70 to 95% purity.

The FOS obtained according to the invention contain short-chain oligomers (scFOS) such as GF2, GF3, GF4 and/or long-chain oligomers (lcFOS) such as GF5 and/or GF6, which are those with the greatest bifidogenic effect, while the former have a greater immunomodulation capacity, which is the great differentiator of the product in question. The larger the chain size, without reaching polymer size, the greater the bifidogenic effect of the product. This is due to the fact that the effectiveness of intermediate chains such as GF5 and GF6 is greater than GF2 and GF3 and much better than long chains of polymers such as inulin and levans, which can be used more as soluble fibers and contribute to the passage faster through intestinal transit and are less bifidogenic or modulators of the intestinal microbiota.

Likewise, smaller oligomers, such as GF2, GF3, have proven to have immunomodulatory activities, acting directly on cells of the immune system present in the intestinal epithelium, such as macrophages, T cells, B cells, among others.

The greater the bifidogenic effect, the greater the increase in the population of good bacteria, such as *lactobacilli* and bifidobacteria, and, therefore, the greater the acidification it will cause in the intestinal tract and the more inhospitable it will become for Salmonella and E. Coli, thus increasing its efficiency as a growth promoter and population controller. In the formulations of the present invention, the GF2 ratio may vary from 10 to 45%, while that of GF3 may vary from 30 to 55%, that of GF4 may vary from 10 to 25%, that of GF5 may vary from 3 to 13% and that of GF6 may vary from 0 to 8%. Preferably, the ratio of FOS oligomers of the present invention may vary between 15 and 40% in GF2, between 35 and 50% in GF3, between 13 and 23% in GF4, between 3 and 10% in GF5 and between 0 and 6% in the GF6.

The biotransformation process used in the present invention has the advantage of controlling, through the synthesis time, the proportion of short-chain oligomers formed. In the invention, the shorter the synthesis time, the greater the proportion of GF2 in relation to GF3 and GF4 and GF5. Each species has its own microbiota that is different from the others. Thus, in the present invention the FOS composition that will be used for birds will be different from the FOS composition that will be used for monogastrics, fish, ruminants or humans. The proportion between short-chain oligomers should vary depending on the purpose of using the FOS.

According to an embodiment of the present invention, the process for producing and purifying fructooligosaccharides employs at least two microbes or yeasts, simultaneously or separately, one of them being *Aureobasidium sp,* the other yeast not being capable of producing invertase extracellular, in a medium containing sucrose, in order to convert sucrose into fructooligosaccharides (FOS), with total consumption of glucose from the medium and evaporation of alcohol or ethanol during drying of the final product.

According to another embodiment of the present invention, the process for producing and purifying fructooligosaccharides employs at least two microbes or yeasts, simultaneously or separately, one of them being the *Aureobasidium sp* and the other yeast *Saccharomyces cerevisiae genetically modified not capable of producing extracellular invertase,* in medium containing sucrose, in order to convert sucrose into fructooligosaccharides (FOS), with total consumption of glucose from the medium and evaporation of alcohol or ethanol during drying of the final product.

According to yet another embodiment of the present invention, the process for producing and purifying fructooligosaccharides, employs an aqueous medium comprising 20% to 60% by mass of sucrose where *Aureobasidium sp* and *Saccharomyces cerevisiae* genetically modified not capable of producing extracellular invertase, in inoculum concentration with a minimum population of $5.10^7$ CFU/ml of each of the two yeasts, remaining at a temperature between 30°C and 35°C, under agitation and aeration, until the glucose present in the medium is completely eliminated and the ethanol is eliminated by drying the final product.

According to yet another embodiment of the present invention, the process for producing and purifying fructooligosaccharides, the inoculum containing the yeasts is prepared from an aqueous medium comprising 10% to 20% total reducing sugars (TRS) inoculated with *Aureobasidium sp* in a volumetric ratio (v/v) of the *medium:Aureobasidium sp of* 1:10 respectively and after its growth, it is inoculated with *Saccharomyces cerevisiae* genetically modified in the same volumetric ratio of 1:10 inoculum:aqueous medium, remaining at a temperature between 32°C and 35°C under agitation and aeration, where, after complete growth of both cultures or yeasts, the inoculum is transferred to a new culture medium containing 10% to 20% TRS in a volumetric ratio (v/v) of 1:10 inoculum:medium respectively, remaining under agitation and aeration, at the same temperature, until complete growth and the minimum population is $5.10^7$ CFU/ml of each yeast, with elimination of ethanol in drying the final product.

According to a preferred embodiment of the invention, first a volume of "9X" liters of aqueous medium containing 10 to 20% total reducing sugars (TRS) is prepared and a volume of "1X" liters of *Aureobasidium* where, after 24 hours of growth, the *Saccharomyces cerevisiae* genetically modified is inoculated, leaving it incubated for another 24 hours, with a temperature between 32 to 35°C, under agitation and aeration, at least. After complete growth of both cultures, the "10X" liters of inoculum culture are transferred to "90X" liters of culture medium containing 10 to 20% TRS (total reducing sugars), incubated at a controlled temperature between 32 to 35° C, under agitation and aeration, until complete growth and a minimum population of 5. $10^7$ CFU/mL of each yeast (*Aureobasidium sp and* genetically modified *Saccharomyces cerevisiae*)*;* then the inoculum is transferred to the aqueous medium of sucrose and/or molasses and/or sugar cane syrup containing 20 to 60% sucrose in the total medium, in the ratio of 10 to 20% of the volume of the aqueous medium, where the synthesis of FOS occurs in a minimum period of 12 hours, but the removal of glucose through the consumption of *genetically modified Sacharomyces* yeast occurs between 12 and 60 hours, a period that varies depending on the initial sucrose concentration of the medium, with temperatures varying between 30 to 35°C.

According to a more preferred form of carrying out the invention, first, a volume of "9X" liters of aqueous medium containing 10 to 20% total reducing sugars (TRS) is prepared and a volume of "1X" liters is inoculated of *Aureobasidium,* and after 24 hours of growth, the genetically modified *Saccharomyces cerevisiae* is inoculated, which is incubated for another 24 hours, with a temperature set between 32 and 35°C, with 120 rpm of agitation and 1 vvm of aeration, in the minimum, after complete growth of both cultures, "10X" liters of inoculum culture are transferred to "90X" liters of culture medium containing 10 to 20% TRS (total reducing sugars), incubated at a controlled temperature between 32 to 35°C, with 120 rpm of agitation and at least 1 vvm of aeration, for 24 to 48 hours, until complete growth and a minimum population of 5. $10^7$ CFU/mL of each yeast *(Aureobasidium sp* and genetically modified *Saccharomyces cerevisiae*); the inoculum is then transferred to an aqueous medium of sucrose and/or molasses and/or sugar cane syrup containing 20 to 60% sucrose in the total medium, in the ratio of 10 to 20% of the volume of the aqueous medium, with the synthesis of FOS occurring in a minimum period of 12 hours, but the removal of glucose through the consumption of *genetically modified Sacharomyces* yeast occurs between 12 and 60 hours, a period that varies depending on the initial sucrose concentration of the medium, with varying temperature between 30 to 35°C.

Purified fructooligosaccharides (FOS) lack glucose and ethanol in the final product.

Below is an example to better illustrate the scope of the invention, however, these examples should not be used for limiting purposes of the invention.

EXAMPLE 1:

Production of fructooligosaccharides (FOS):

An *Aureobasidium sp* strain, responsible for the synthesis of FOS, and a *Saccharomyces cerevisiae* PE-2 strain that does not produce extracellular invertase were used in one single reactor. To do this, first, 200L of aqueous medium containing 15% total reducing sugars (TRS) was prepared, 20L of Aureobasidium was inoculated and after 24 hours of growth, genetically modified *Saccharomyces cerevisiae PE-2* was inoculated, which was incubated for another 24 hours, with a temperature set between 33°C, with 120 rpm of agitation and at least 1 vvm of aeration.

After complete growth of both cultures, the 200L of inoculum were transferred to 2000L of culture medium containing 15% TRS (total reducing sugars), and incubated at a controlled temperature of 33°C, with 120 rpm of shaking and with at least 1 vvm of aeration, for 36 hours, until complete growth and a minimum population of 5. $10^7$ CFU/mL of each yeast *(Aureobasidium sp* and *Saccharomyces cerevisiae*).

After growth, the inoculum was transferred to the aqueous medium of sucrose sugar cane syrup containing 40% sucrose in the total medium, in the ratio of 15% of the inoculum volume.

The synthesis of FOS occurred in a minimum period of 12 hours. However, the removal of glucose through the consumption of *Sacharomyces* yeast occurred after 40 hours, a period that varied depending on the initial sucrose concentration of the medium, at a temperature of 33°C, for a period of 40 hours.

The ethyl alcohol produced was 100% evaporated during spray drying so that the final product now contains high purity.

The FOS produced presented only short-chain oligomers (GF2, GF3 and GF4, GF5 and/or GF6), which are those with the greatest bifidogenic effect, in the following proportions by mass of oligomers: 24% in GF2, 35% in GF3, between 32% in GF4, between 7% in GF5 and between 2% in GF6.

Application of FOS obtained in cats:

An experiment was carried out with cats consuming different types of prebiotics, including the FOS of the present invention (yFOS) and the prior art FOS, produced by Ingredrion (scFOS), designed in randomized blocks, with the period being the blocking factor. The experiment consisted of six treatments, repeated in three periods, with 12 cats in each period (two cats per treatment), totaling six repetitions per treatment (n=36 total).

The objective of this experiment was to demonstrate that the inclusion of the prebiotics FOS, GOS and inulin in the diet of cats would modify the concentrations of fecal fermentation products, resulting from greater intestinal fermentation of organic matter and, as a consequence, the results of apparent digestibility of the diet would be modified. Furthermore, the objective was to show that there are significant differences in performance between the FOS of the present invention (yFOS) and the FOS of the prior art, produced by the company Ingredion, (scFOS).

Six experimental diets were formulated, one being a Negative Control (NC) diet, without the addition of any prebiotic and another five containing, respectively, 0.8% FOS of the present invention, from the company Yessinergy (yFOS); 0.8% GOS (GOS); 0.8% inulin (INU); 0.8% FOS from prior art, produced by the company Ingredion (scFOS) and; 0.8% of a combination of yFOS and inulin (yFOS+INU). The inclusion of prebiotics was made to replace cellulose, as it is a fiber, however, not fermentable for cats. All diets were formulated based on the recommendations of FEDIAF (2018), the acronym of the European Federation of the Pet Food Industry.

At the end of the digestibility test, all cats were kept for an additional period, until completing 21 days of total period, for the daily collection of fresh feces, up to 15 minutes after defecation, to determine the pH, volatile fatty acids (VFA), fecal ammonia nitrogen and fecal lactate.

To measure fecal pH, two grams of fresh feces (collected within 15 minutes after defecation) were diluted in 6 mL of milliQ water and the pH was evaluated by using a precision digital pH meter.

To determine the concentration of VFA (acetic, propionic, butyric, isobutyric, isovaleric, valeric, 4-methyl-valeric, hexanoic and heptanoic) and fecal ammonia nitrogen, 5 grams of fresh feces were collected and quickly homogenized and mixed with 15 mL of 16% formic acid solution (1:3 w/v). This mixture remained refrigerated and was centrifuged three times at 4,500 G, for 15 minutes each, using the supernatant and discarding the sediment. To measure VFA, the samples were identified and stored in a freezer (-15°C) and subsequently submitted for analysis at the Chemistry Laboratory, at the State University of Maringá. The determination of VFA in feces was determined by gas chromatography.

Ammonia nitrogen in feces was determined by adapting the Kjeldahl method. The extracts prepared for VFA measurement were used. The extracts were thawed at room temperature and 2 mL aliquots were transferred to a test tube, diluted in 13 mL of distilled water and subjected to distillation in a nitrogen distiller (Tecnal T-036/1 Piracicaba, Brazil). Distillation was carried out with 5 mL of potassium hydroxide KOH 2 mol/L and nitrogen received in an Erlenmeyer flask with 10 mL of solution (boric acid 0.97 N). Next, each sample was titrated with HCL (0.005 N).

The variables measured in each experiment were previously analyzed for normality and equality of variance. The apparent digestibility coefficients between treatments were compared by using the Tukey Test, at 5% probability. For fermentation products (ammonia, SCFA and BCFA), pH and fecal DM, considering that these present an interdependence between themselves, multivariate statistics were applied, through the analysis of canonical correlations, also considering this as significant, when the Analysis of Multivariate Variance (MANOVA) presented a probability value of less than 5% for the null hypothesis.

Below, Table 4, on fermentative parameters in feces (short-chain fatty acids (mmol/kg Fecal MS), where NC = negative control; yFOS = fructooligosaccharides according to the present invention; GOS = galactooligosaccharides; INU = inulin; scFOS = FOS produced by Ingredion; FOS + INU = FOSy (FOS according to the present invention) associated with INU:

TABLE 4

|  | NC | yFOS | GOS | INU | scFOS | INU+FOS |
|---|---|---|---|---|---|---|
| Acetic | 26.8 | 26.1 | 28.1 | 20.4 | 17.1 | 22.6 |
| Propionic | 8.0 | 7.0 | 9.1 | 5.2 | 6.0 | 8.2 |
| Butyric | 4.4 | 4.5 | 4.1 | 2.7 | 4.1 | 4.9 |
| Isobutyric | 1.6 | 1.8 | 1.7 | 1.5 | 1.5 | 1.7 |
| Isovaleric | 1.8 | 2.3 | 1.8 | 1.6 | 1.8 | 1.8 |
| Valeric | 3.2 | 3.7 | 2.5 | 2.4 | 2.8 | 3.1 |
| 4-methyl-valeric | 0.49 | 0.67 | 0.31 | 0.4 | 0.22 | 0.24 |
| Hexanoic | 0.24 | 0.35 | 0.24 | 0.25 | 0.22 | 0.24 |
| Heptanoic | 1.9 | 2.2 | 2.1 | 1.7 | 1.8 | 1.9 |
| Total VFA | 39.2 | 37.6 | 41.3 | 28.3 | 27.1 | 35.7 |
| Total SCFA | 7.1 | 8.6 | 6.2 | 5.9 | 6.3 | 6.9 |
| Ammonia (mq/kq DM) | 1.8 | 2.5 | 2.1 | 1.7 | 1.9 | 2.1 |
| Lactate (mmol/kq DM) | 11.6 | 12.4 | 25.6 | 14.5 | 16.9 | 18.7 |
| Fecal pH | 6.2 | 6.1 | 6.0 | 6.1 | 5.9 | 6.1 |

(continued)

|  | NC | yFOS | GOS | INU | scFOS | INU+FOS |
|---|---|---|---|---|---|---|
| Fecal MS (%) | 40.4 | 35.6 | 34.9 | 39.0 | 39.9 | 36.4 |

As shown in Table 4, the formation of intestinal fermentation products such as lactate, propionate and acetate were more correlated with the inclusion of GOS in the diets and the others (butyrate, BCFA and ammonia) showed a greater correlation with the inclusion of yFOS (present invention), demonstrating that these two treatments provided greater intestinal fermentation than the others. Fecal pH was inversely related to fermentation products, as expected.

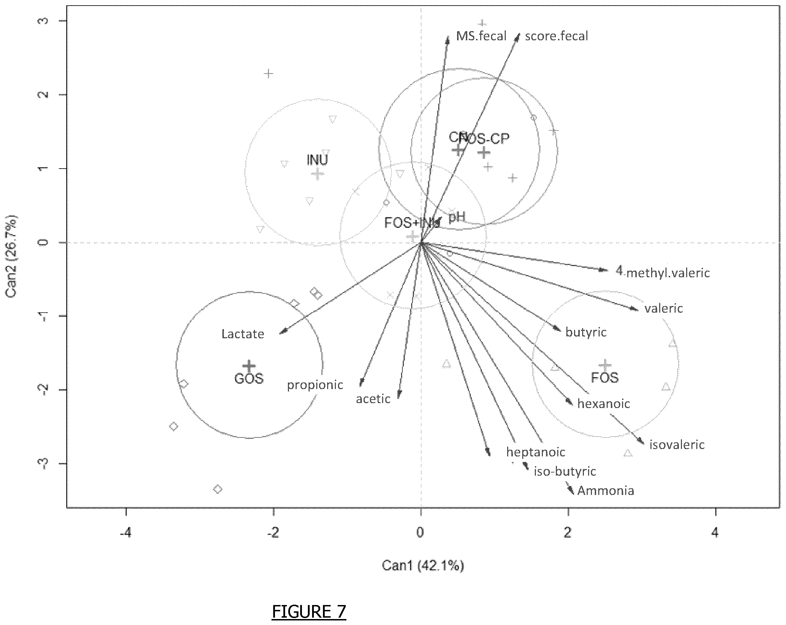

Figure 7 attached represents the Canonical Correlation for the fermentative and fecal quality parameters between the Negative Control (NC) and prebiotic-containing treatments.

Although the higher ammonia content and greater softness of feces are not desirable in domestic cat feces, the objective of the experiment was to show the greater general bifidogenic effect of the FOS of the present invention, when compared to other prebiotics available on the market. In this aspect, the high values of total volatile fatty acids (total VFAs) and, mainly, of total short-chain fatty acids (total SCFAs), show the superior performance of the product of the present invention. An even more important indicator that there was greater fermentation, throughout the large intestine, in the yFOS and yFOS+INU treatments, was their greater production of butyric acid. Currently, butyric acid is the most desired by nutritionists.

In Table 5 below, the blood parameters related to the phagocytosis of monocytes and granulocytes (per $\mu$L of blood) are presented, where NC = negative control; yFOS = fructooligosaccharides according to the present invention; GOS = galactooligosaccharides; INU = inulin; scFOS = FOS produced by Ingredion; FOS + INU = FOSy (FOS according to the present invention) associated with INU:

TABLE 5

|  | NC | yFOS | GOS | INU | scFOS | FOS+INU |
|---|---|---|---|---|---|---|
| Phagocytic Monocytes |  |  |  |  |  |  |
| % | 56.9 | 65.5 | 60.6 | 54.5 | 57.5 | 47.4 |
| N | 160,384 | 269,163 | 214,144 | 282,573 | 321,787 | 268,610 |
| Phagocytic Granulocytes |  |  |  |  |  |  |
| % | 1.4 | 1.6 | 1.5 | 1.1 | 1.2 | 0.7 |
| N | 2,683 | 3,837 | 4,514 | 6,399 | 4,682 | 5,305 |
| Total Leukocytes |  |  |  |  |  |  |
| N | 304,048 | 398,000 | 349,480 | 543,563 | 504,288 | 648,356 |

Table 5 shows that in treatment with yFOS, there was the highest concentration of monocytes and phagocytic granulocytes, as well as a low concentration of total leukocytes, which is also highly desirable. This demonstrates the superiority of yFOS, the product of the present invention, in modulating the immune system of cats. It also indicates that yFOS increased the protagonism of the cellular immune system and reduced that of the humoral immune system, i.e., it had an anti-inflammatory effect.

Likewise, Table 6 contains the results regarding fecal IgA concentration, where NC = negative control; yFOS = fructooligosaccharides according to the present invention; GOS = galactooligosaccharides; INU = inulin; scFOS = FOS produced by Ingredion; FOS + INU = FOSy (FOS according to the present invention) associated with INU:

TABLE 6

|  | NC | yFOS | GOS | INU | scFOS | FOS+INU |
|---|---|---|---|---|---|---|
| Fecal IqA ($\mu$g/mL) | 1.06 | 3.09 | 4.76 | 5.95 | 7.32 | 2.06 |

The results shown in Table 6 reaffirm the results shown in tables 4 and 5, demonstrating the superiority of treatment with yFOS, a product of the present invention, with regard to its superiority in terms of bifidogenic effect and modulation of the immune system, when compared to other treatments, including treatment with scFOS, which represents an important commercial brand of FOS in prior art. Reaffirming that yFOS increased the cellular immune system and reduced the humoral immune system, i.e., it had an anti-inflammatory effect.

**Claims**

1.  Process for the production and purification of fructooligosaccharides **characterized by** employing at least two microbes or yeasts, simultaneously or separately, with one of the microbes or yeasts not being capable of producing extracellular invertase, in a medium containing sucrose, in order to convert sucrose into fructooligosaccharides (FOS), with total consumption of glucose from the medium and evaporation of alcohol or ethanol during drying of the final product.

2.  Process for production and purification of fructooligosaccharides, according to claim 1, **characterized by** the FOS obtained presents long-chain oligomers GF5 and GF6, and a larger average size of the other short-chain oligomers GF2, GF3 and GF4.

3.  Process for production and purification of fructooligosaccharides, according to claim 1, **characterized by** the ratio of oligomers in the fructooligosaccharides (FOS) comprises, in relation to the total mass, GF2 from 10 to 45%, GF3 from 30 to 55%, GF4 from 10 to 25%, GF5 from 3 to 13% and GF6 from 0 to 8%.

4.  Process for production and purification of fructooligosaccharides, according to claim 3, **characterized by** the proportions of FOS oligomers vary in mass in relation to the total mass: between 15 and 40% GF2, between 35 and 50% GF3, between 13 and 23% of GF4, between 3 and 10% of GF5 and between 0 and 6% of GF6.

5.  Process for the production and purification of fructooligosaccharides, according to claim 1, **characterized by** using as sources of sucrose, sugars from sugar cane, beetroot, sugar cane syrup and molasses.

6.  Process for production and purification of fructooligosaccharides, according to claims 1 or 5, **characterized by** the medium contains 20 to 60% by mass of sucrose.

7.  Process for production and purification of fructooligosaccharides, according to claim 1, **characterized by** one of the microbes or yeasts is *Aureobasidium sp.*

8.  Process for production and purification of fructooligosaccharides, according to claim 1, **characterized by** one of the microbes or yeasts being genetically modified *Saccharomyces cerevisiae.*

9.  Process for production and purification of fructooligosaccharides, according to claim 1, **characterized by** the genetically modified *Saccharomyces cerevisiae* is of a wild strain called PE-2, isolated from a Brazilian industrial sugar and alcohol environment, which was subjected to a simple deletion of the gene related to invertase production.

10. Process for production and purification of fructooligosaccharides, according to claim 1, **characterized by** the genetically modified yeast *Saccharomyces cerevisiae* selectively consumes only glucose and fructose and does not consume sucrose and FOS, i.e., the oligomers with type $\alpha$ linkage,1-2 and $\beta$,2-1.

11. Process for production and purification of fructooligosaccharides, according to claim 8, **characterized by** gene manipulation having been applied to chromosome 9.

12. Process for the production and purification of fructooligosaccharides, according to claim 1, **characterized by** employing at least two microbes or yeasts, simultaneously or separately, one of them being *Aureobasidium sp* and the other yeast is not capable of producing extracellular invertase, in medium containing sucrose, in order to convert sucrose into fructooligosaccharides (FOS), with total consumption of glucose from the medium and evaporation of alcohol or ethanol by evaporation during drying of the final product.

13. Process for production and purification of fructooligosaccharides, according to claim 12, **characterized by** employing at least two microbes or yeasts, simultaneously or separately, one of them being *Aureobasidium sp* and the other *genetically modified Saccharomyces cerevisiae yeast not capable of producing invertase extracellular,* in a medium containing sucrose, in order to convert sucrose into fructooligosaccharides (FOS), with total consumption of glucose from the medium and evaporation of alcohol or ethanol during drying of the final product.

14. Process for production and purification of fructooligosaccharides, according to any of the previous claims, **characterized by** employing an aqueous medium comprising 20% to 60% by mass of sucrose where *Aureobasidium sp* and genetically modified *Sacchaormyces cerevisiae* not capable of producing invertase are inoculated extracellular, in an inoculum concentration presenting a minimum population of $5,10^7$ CFU/ml of each of the two yeasts, remaining at a temperature between 30°C and 35°C, under agitation and aeration, until total elimination of the glucose present in the medium and elimination of ethanol by evaporation during the drying the final product.

15. Process for production and purification of fructooligosaccharides, according to any of the previous claims, **characterized by** the inoculum containing the yeasts is prepared from an aqueous medium comprising 10% to 20% total reducing sugars (TRS) inoculated with *Aureobasidium sp* in a volumetric ratio (v/v) of medium: *Aureobasidium sp of* 1:10 respectively and after its growth, it was inoculated with genetically modified *Saccharomyces cerevisiae* in the same volumetric ratio of 1:10 inoculum: medium, remaining at a temperature between 32°C and 35°C under agitation and aeration, and after complete growth of both cultures or yeasts, the inoculum is transferred to a new culture medium containing 10% to 20% TRS in a volumetric ratio (v/v) of 1:10 inoculum:medium respectively, remaining under agitation and aeration, at the same temperature, until complete growth and the minimum population is $5,10^7$ CFU/ml of each yeast.

16. Process for production and purification of fructooligosaccharides, according to any of the previous claims, **characterized by** first preparing a volume of "9X" liters of aqueous medium containing 10 to 20% total reducing sugars (TRS) and inoculating it a volume of "1X" liters of *Aureobasidium* and after 24 hours of growth, the genetically modified *Saccharomyces cerevisiae* is inoculated, remaining incubated for another 24 hours, with a temperature between 32 to 35°C, under agitation and aeration, at least, after complete growth of both cultures "10X" liters of inoculum culture are transferred to "90X" liters of culture medium containing 10 to 20% TRS (total reducing sugars), incubated at a controlled temperature between 32 to 35°C, under agitation and aeration, until complete growth and a minimum population of $5.10^7$ CFU/mL of each yeast (*Aureobasidium sp* and genetically modified *Saccharomyces cerevisiae*); then the inoculum is transferred to the aqueous medium of sucrose and/or molasses and/or sugar cane syrup containing 20 to 60% sucrose in the total medium, in the ratio of 10 to 20% of the volume of the aqueous medium, where the synthesis of FOS occurs in a minimum period of 12 hours, but the removal of glucose through the consumption of *genetically modified Sacharomyces* yeast occurs between 12 and 60 hours, a period that varies depending on the initial sucrose concentration of the medium, with temperatures varying between 30 to 35°C.

17. Product obtained by process according to claims 1 to 16, **characterized by** comprising purified fructooligosaccharides (FOS), with the absence of glucose and ethanol in the final product.

18. Product, according to claim 17, **characterized by** the fructooligosaccharides (FOS) obtained present long-chain oligomers GF5 and GF6, and a larger average size of the other short-chain oligomers GF2, GF3 and GF4.

19. Product, according to claims 17 or 18, **characterized by** the mass ratio of GF2 varies from 10 to 45%, that of GF3 varies from 30 to 55%, that of GF4 varies from 10 to 25%, that of GF5 varies from 3 to 13% and GF6 varies from 0 to 8%.

20. Product, according to claim 19, **characterized by** the mass ratios of FOS oligomers vary between 15 and 40% GF2, between 35 and 50% GF3, between 13 and 23% GF4, between 3 and 10% GF5 and between 0 and 6% GF6.

21. Product, according to claims 17 to 20, **characterized by** comprising fructooligosaccharides (FOS) and 3 to 10% by mass in relation to the total mass of drying supporting additives known to the person skilled in the art.

## TRADITIONAL PROCESS

fructosyltransferase

Food grade
Activated charcoal

**Sucrose** ⟶ Conversion

FOS >55-60%

⟶ **Desalination**

Food grade
Activated charcoal

**Concentration in vacuum** ⟶ Purification chromatographic ⟶ **FOS 95%** ⟶ **Discoloration**

**Concentration in vacuum** ⟶ FOS 95% syrup / Spray dry ⟶ **FOS 95% purity powder**

FIGURE 1

EP 4 495 224 A1

# YES BIOTRANSFORMATION PROCESS

Simultaneous synthesis and
purification process

Conversion

**Sucrose** ⟶ FOS>80%-90% ⟶

Spray dry ⟶ FOS 80-90% powder

FIGURE 2

FIGURE 3

EP 4 495 224 A1

FIGURE 4

FIGURE 5

FIGURE 6

FIGURE 7

**EP 4 495 224 A1**

<div style="text-align:center">

**INTERNATIONAL SEARCH REPORT**

</div>

| | International application No. |
|---|---|
| | **PCT/BR2023/050086** |

**A.    CLASSIFICATION OF SUBJECT MATTER**

C12N 1/00 (2006.01)i

According to International Patent Classification (IPC) or to both national classification and IPC

**B.    FIELDS SEARCHED**

Minimum documentation searched (classification system followed by classification symbols)

C12P 19/24; C12N 1/00; C12N 15/55; C12N 9/26

Documentation searched other than minimum documentation to the extent that such documents are included in the fields searched

Bases de Patente do INPI

Electronic data base consulted during the international search (name of data base and, where practicable, search terms used)

PubMed; Derwent Innovation; Espacenet

**C.    DOCUMENTS CONSIDERED TO BE RELEVANT**

| Category* | Citation of document, with indication, where appropriate, of the relevant passages | Relevant to claim No. |
|---|---|---|
| Y | US 2021230656 A1 (DSM IP ASSETS BV [NL]) 29 July 2021 (2021-07-29)<br>    The whole document. | 1-21 |
| Y | WO 2012007481 A2 (UNIV GENT [BE]) 19 January 2012 (2012-01-19)<br>    Example 33 and example 34. Emphasis on the phrase "Because sucrose phosphorylase competes for sucrose with invertase, the genes coding for invertase activity are knocked out." | 1-21 |
| T | Cosmi et al. Fructooligosaccharides: From Breast Milk Components to Potential Supplements. A Systematic Review. Adv Nutr. 2022 Feb 1;13(1):318-327. doi: 10.1093/advances/nmab102. Abstract: "Breast milk is the optimal food choice for infant growth and development. Among breast milk components, fructooligosaccharides (FOSs) are being actively studied because of their role in microbiota development." | 1-21 |

☐ Further documents are listed in the continuation of Box C.        ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **04 August 2023** | **07 August 2023** |

| Name and mailing address of the ISA/BR | Authorized officer |
|---|---|
| **National Institute of Industrial Property (Brazil)**<br>**Rua Mayrink Veiga, 9, 6º andar, CEP 20.090-910 Rio de Janeiro – RJ**<br>**Brazil** | **Amanda FERREIRA** |
| Telephone No. **(55 21) 3037-3742, 3037-3984** | Telephone No. **+55 21 3037 4528 - 3037 3319** |

Form PCT/ISA/210 (second sheet) (July 2022)

INTERNATIONAL SEARCH REPORT
Information on patent family members

International application No.

**PCT/BR2023/050086**

| Patent document cited in search report | | | Publication date (day/month/year) | Patent family member(s) | | | Publication date (day/month/year) |
|---|---|---|---|---|---|---|---|
| US | 2021230656 | A1 | 29 July 2021 | BR | 112020023639 | A2 | 17 February 2021 |
| | | | | CN | 112154150 | A | 29 December 2020 |
| | | | | EP | 3799594 | A1 | 07 April 2021 |
| | | | | EP | 3799594 | A4 | 15 June 2022 |
| | | | | JP | 2021524238 | A | 13 September 2021 |
| | | | | KR | 20210013138 | A | 03 February 2021 |
| | | | | WO | 2019226431 | A1 | 28 November 2019 |
| WO | 2012007481 | A2 | 19 January 2012 | WO | 2012007481 | A3 | 15 March 2012 |
| | | | | AU | 2011278315 | A1 | 24 January 2013 |
| | | | | AU | 2011278315 | B2 | 28 May 2015 |
| | | | | CA | 2804713 | A1 | 19 January 2012 |
| | | | | CA | 2804713 | C | 27 April 2021 |
| | | | | CN | 103025874 | A | 03 April 2013 |
| | | | | CN | 107603935 | A | 19 January 2018 |
| | | | | CN | 113736721 | A | 03 December 2021 |
| | | | | DE | 202011111144 | U1 | 10 February 2021 |
| | | | | EP | 2593549 | A2 | 22 May 2013 |
| | | | | EP | 3235907 | A1 | 25 October 2017 |
| | | | | EP | 3235907 | B1 | 21 June 2023 |
| | | | | JP | 2013535962 | A | 19 September 2013 |
| | | | | JP | 5998130 | B2 | 28 September 2016 |
| | | | | JP | 2017018093 | A | 26 January 2017 |
| | | | | JP | 6310964 | B2 | 11 April 2018 |
| | | | | JP | 2018121641 | A | 09 August 2018 |
| | | | | JP | 6599498 | B2 | 30 October 2019 |
| | | | | MX | 2013000460 | A | 03 June 2013 |
| | | | | MX | 353506 | B | 17 January 2018 |
| | | | | MX | 2020011690 | A | 10 December 2020 |
| | | | | MX | 2020011691 | A | 10 December 2020 |
| | | | | MX | 2020011693 | A | 10 December 2020 |
| | | | | MX | 2020011713 | A | 08 January 2021 |
| | | | | MX | 2020011714 | A | 08 January 2021 |
| | | | | MX | 2020011715 | A | 08 January 2021 |
| | | | | MY | 185012 | A | 30 April 2021 |
| | | | | NZ | 605093 | A | 30 May 2014 |
| | | | | SG | 186836 | A1 | 28 February 2013 |
| | | | | SG | 10201504733X | A | 30 July 2015 |
| | | | | US | 2013122553 | A1 | 16 May 2013 |
| | | | | US | 9701992 | B2 | 11 July 2017 |
| | | | | US | 2017016038 | A1 | 19 January 2017 |
| | | | | US | 10570430 | B2 | 25 February 2020 |
| | | | | US | 2020140908 | A1 | 07 May 2020 |
| | | | | US | 11384374 | B2 | 12 July 2022 |
| | | | | US | 2021017558 | A1 | 21 January 2021 |

Form PCT/ISA/210 (patent family annex) (July 2022)

23

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H04293494 B

**Non-patent literature cited in the description**

- **RYAN OW** ; **SKERKER JM** ; **MAURER MJ et al.** Selection of chromosomal DNA libraries using a multiplex CRISPR system. *eLife*, 2014, vol. 3, e03703

- **JAKOCIUNAS T** ; **BONDE I** ; **HERRGARD M et al.** Multiplex metabolic pathway engineering using CRISPR/Cas9 in Saccharomyces cerevisiae. *Metab Eng*, 2015, vol. 28, 213-22